# EUROPEAN PATENT SPECIFICATION

(11) **EP 4 401 690 B1**
(45) Date of publication and mention of the grant of the patent: **07.10.2026**
(21) Application number: 22786107.7
(22) Date of filing: 15.09.2022
(51) Int. Cl.: A61F 9/009

(54) **PATIENT INTERFACES FOR COOLING EYE TISSUE**
PATIENTENSCHNITTSTELLEN ZUR KÜHLUNG VON AUGENGEWEBE
INTERFACES DE PATIENT POUR REFROIDIR UN TISSU OCULAIRE

(30) Priority: 17.09.2021 US 202163245480 P
(43) Date of publication of application: 24.07.2024
(73) Proprietor: Alcon Inc., 1701 Fribourg (CH)
(72) Inventor: ABRAHAM, Mario, 91058 Erlangen (DE); WITTNEBEL, Michael, 91058 Erlangen (DE)
(74) Representative: Mooser, Sebastian Thomas
(86) International application number: PCT/IB2022/058739
(87) International publication number: WO 2023/042130

(56) References cited:
- WO-A2-2006/128038
- WO-A2-2020/093060
- US-A- 5 616 139
- US-A- 5 779 696
- US-A1- 2009 137 989
- US-A1- 2020 306 080

## Description

### TECHNICAL FIELD

The present disclosure relates generally to patient interfaces for ophthalmic laser surgical systems, and more particularly to patient interfaces that cool eye tissue.

### BACKGROUND

Ophthalmic laser surgery uses laser pulses to irradiate, shape, and/or cut tissue of the eye. During surgery, laser pulses are directed towards the eye. As the laser pulses hit a location of the eye, they increase the temperature at the location. If the temperature exceeds a damage threshold, the eye may suffer temporary or permanent damage. For example, the IEC standard 60601-1 sets 41°C as the maximum temperature of the tissue. Accordingly, it is important to cool the tissue of the eye. Patient interfaces with cooling systems are described in WO 2020/093060.

### BRIEF SUMMARY

The scope of present invention is defined by the set of claims. Methods described are not part of the claimed invention. In certain embodiments, a patient interface for an ophthalmic laser system includes an interface portion and an attachment portion. The interface portion includes a transmissive portion and an interface wall. The transmissive portion allows a laser beam through to the cornea of an eye to perform an ophthalmic procedure, and the interface wall is disposed outwardly from the transmissive portion. The attachment portion couples the interface portion to a region of the cornea to allow the laser beam through to the cornea to perform the ophthalmic procedure. The attachment portion also decreases the temperature of the region during the ophthalmic procedure.

Embodiments may include none, one, some, or all of the following features: The attachment portion includes one or more channels, and decreases the temperature of the region by receiving fluid through the one or more channels to transfer heat away from the region. The attachment portion includes a receptacle configured to receive a cold core, and decreases the temperature of the region by allowing the cold core to transfer heat away from the region. The attachment portion includes a heat pump configured to transfer heat, and decreases the temperature of the region by transferring heat, with the heat pump, away from the region. The attachment portion has an annular shape that receives the interface wall of the interface portion to removably couple the interface portion to the attachment portion. The interface portion and the attachment portion formed as formed as one piece.

In certain embodiments, a patient interface for an ophthalmic laser system includes an interface portion and an attachment portion. The interface portion includes a transmissive portion and an interface wall. The transmissive portion allows a laser beam through to the cornea of an eye to perform an ophthalmic procedure. The transmissive portion also decreases the temperature of the region during the ophthalmic procedure. The interface wall is disposed outwardly from the transmissive portion. The attachment portion couples the interface portion to a region of the cornea to allow the laser beam through to the cornea to perform the ophthalmic procedure.

Embodiments may include none, one, some, or all of the following features: The transmissive portion includes first and second layers. The first layer transfers heat in a first direction. The second layer is disposed outwardly from the first layer, and transfers heat in a second direction substantially opposite to the first direction. The attachment portion includes one or more channels, and decreases the temperature of the region by receiving fluid through the one or more channels to transfer heat away from the region. The attachment portion includes a receptacle configured to receive a cold core, and decreases the temperature of the region by allowing the cold core to transfer heat away from the region. The attachment portion includes a heat pump configured to transfer heat, and decreases the temperature of the region by transferring heat, with the heat pump, away from the region. The attachment portion has an annular shape that receives the interface wall of the interface portion to removably couple the interface portion to the attachment portion. The interface portion and the attachment portion formed as formed as one piece.

In certain embodiments, a method for cooling a region of the cornea of an eye during an ophthalmic laser procedure includes coupling a patient interface outwardly from the region of the cornea for the ophthalmic laser procedure. The patient interface includes an interface portion and an attachment portion. The interface portion allows a laser beam through to the cornea to perform the ophthalmic laser procedure at the cornea. The attachment portion couples the interface portion to the region of the cornea. The method includes transferring, by the patient interface, heat away from the region of the cornea.

Embodiments may include none, one, some, or all of the following features: The interface portion of the patient interface transfers heat away from the region of the cornea. The attachment portion of the patient interface transfers heat away from the region of the cornea. The method further includes cooling at least a part of the patient interface prior to coupling the patient interface outwardly from the region of the cornea. The method further includes cooling the interface portion of the patient interface prior to coupling the patient interface outwardly from the region of the cornea. The method further includes cooling the attachment portion of the patient interface prior to coupling the patient interface outwardly from the region of the cornea.

In certain embodiments, a patient interface for an ophthalmic laser system includes an interface portion and an attachment portion. The interface portion includes a transmissive portion and an interface wall. The transmissive portion allows a laser beam through to the cornea of an eye to perform an ophthalmic procedure. The interface wall is disposed outwardly from the transmissive portion. The attachment portion couples the interface portion to a region of the cornea to allow the laser beam through to the cornea to perform the ophthalmic procedure. The attachment portion and interface portion decrease the temperature of the region during the ophthalmic procedure by: receiving fluid through one or more channels to transfer heat away from the region; comprising a receptacle that receives a cold core and allows the cold core to transfer heat away from the region; and/or comprising a heat pump that transfers heat away from the region.

### BRIEF DESCRIPTION OF THE DRAWINGS

FIGURE 1 illustrates an example of an ophthalmic surgical system with a patient interface that decreases the temperature of tissue at a surgical site of an eye, according to certain embodiments;
FIGURE 2 illustrates an example of a patient interface that may be used to stabilize the position of an eye relative to the ophthalmic surgical system of FIGURE 1, according to certain embodiments;
FIGURE 3 illustrates an example of an attachment portion of the patient interface of FIGURE 2 that transfers heat away from the cornea;
FIGURE 4 illustrates another example of an attachment portion of the patient interface of FIGURE 2 that transfers heat away from the cornea;
FIGURE 5 illustrates another example of an attachment portion of the patient interface of FIGURE 2 that transfers heat away from the cornea;
FIGURES 6 , 7A, 7B, 7C and 7D illustrate examples of an interface portion of the patient interface of FIGURE 2 that transfer heat away from the cornea: FIGURE 6 shows a side view of a channel; FIGURES 7A, 7B, and 7C show top views of channels; and FIGURE 7D shows a perspectival view of a channel;
FIGURE 8 illustrates another example of an interface portion of the patient interface of FIGURE 2 that transfers heat away from the cornea;
FIGURE 9 illustrates another example of an interface portion of the patient interface of FIGURE 2 that transfers heat away from the cornea;
FIGURE 10 illustrates an example of a method for cooling the cornea during an ophthalmic procedure that may be used with the patient interface of FIGURE 2, according to certain embodiments; and
FIGURE 11 illustrates another example of a method for cooling the cornea during an ophthalmic procedure that may be used with the patient interface of FIGURE 2, according to certain embodiments.

### DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

In certain embodiments, a patient interface can be used to cool eye tissue before, during, and/or after an ophthalmic procedure in order to avoid damaging the eye. In the embodiments, the patient interface includes an interface portion and an attachment portion. The interface portion allows a laser beam through to the cornea to perform an ophthalmic procedure, and the attachment portion couples the interface portion to the cornea. The interface portion and/or attachment portion may have structures that cool the cornea before, during, and/or after the ophthalmic procedure.

Cooling eye tissue with the patient interface may provide advantages in certain situations. First, the patient interface may provide flexibility to make adjustments in surgery parameters that increase tissue temperature. For example, the patient interface may allow for higher laser shot frequencies or pulse energies, which may decrease treatment time. As another example, temperature-related treatment pauses may possibly be reduced or omitted, which may avoid extending treatment time. Second, the patient interface may reduce temperature-related side effects and other problems, which may improve the safety and effectiveness of the treatment. Third, the patient interface may allow for treatment in more difficult ambient conditions, e.g., in non-air-conditioned areas in a very hot regions.

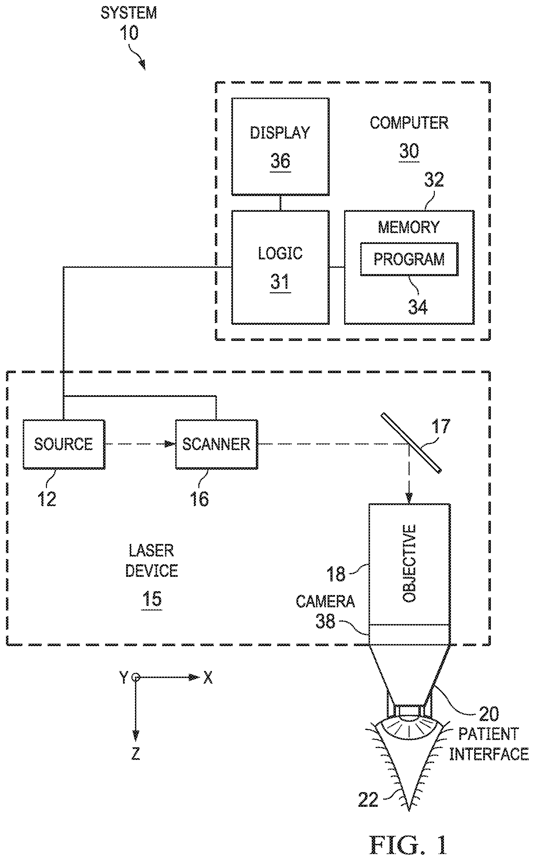

FIGURE 1 illustrates an example of an ophthalmic surgical system 10 with a patient interface 20 that decreases the temperature of tissue at a surgical site of an eye 22, according to certain embodiments. In the illustrated example, system 10 includes a laser device 15, a patient interface 20, a camera 38, and a control computer 30, coupled as shown. Computer 30 includes logic 31, a memory 32 (which stores a computer program 34), and a display 36, coupled as shown. Laser device 15 includes controllable components, such as a laser source 12, a scanner 16, one or more optical elements 17, and a focusing objective 18, coupled as shown. Patient interface 20 is coupled as shown.

Turning to the parts of system 10, laser source 12 may be any suitable source that generates a laser beam, e.g., an excimer laser or an ultrashort pulse laser such as a femtosecond laser. An ultrashort pulse refers to a light pulse that has a duration less than a nanosecond, such as on the order of picoseconds, femtoseconds, or attoseconds. The laser beam may have any suitable wavelength, such as a wavelength in the range of 300 to 1500 nanometers (nm). In some embodiments, the focal point of the laser beam may create a laser-induced optical breakdown (LIOB) in tissue (e.g., corneal tissue) to yield a photodisruption in the tissue. In other embodiments, the focal point of the laser beam may ablate the tissue to remove portions of the tissue.

Scanner 16 transversely and longitudinally directs the focal point of the laser beam. The longitudinal direction refers to the direction of the laser beam propagation, also known as the z-direction. The transverse direction refers to directions orthogonal to the direction of beam propagation, also known as the xy-directions. Scanner 16 may transversely direct the laser beam in any suitable manner. For example, scanner 16 may include a pair of galvanometrically-actuated scanner mirrors that can be tilted about mutually perpendicular axes. As another example, scanner 16 may include an electro-optical crystal that can electro-optically steer the laser beam. Scanner 16 may longitudinally direct the laser beam in any suitable manner. For example, scanner 16 may include a longitudinally adjustable lens, a lens of variable refractive power, or a deformable mirror that can control the z-position of the beam focus. The components of scanner 16 may be arranged in any suitable manner along the beam path, e.g., in the same or different modular units.

One (or more) optical elements 17 direct the laser beam towards focusing objective 18. An optical element 17 can act on (e.g., transmit, reflect, refract, diffract, collimate, condition, shape, focus, modulate, and/or otherwise act on) a laser beam. Examples of optical elements include a lens, prism, mirror, diffractive optical element (DOE), holographic optical element (HOE), and spatial light modulator (SLM). In the example, optical element 17 is a mirror. Focusing objective 18 focuses the focal point of laser beam through the patient interface 20 towards a point of eye 22. In the example, focusing objective 18 is an objective lens, e.g., an f-theta objective.

Camera 38 records images of the movement of eye 22. Examples of camera 38 include a video, optical coherence tomography (OCT), or eye-tracking camera. Camera 38 delivers image data, which represent recorded images of the eye 22, to computer 30. Computer 30 may perform image processing on the image data to identify features of the eye to align the eye.

Computer 30 controls controllable components (e.g., laser source 12, scanner 16, optical elements 17, and/or focusing objective 18) in accordance with computer program 34. Computer program 34 includes computer code that instructs the controllable components to focus the laser beam at a region of the cornea and to photodisrupt at least a portion of the region.

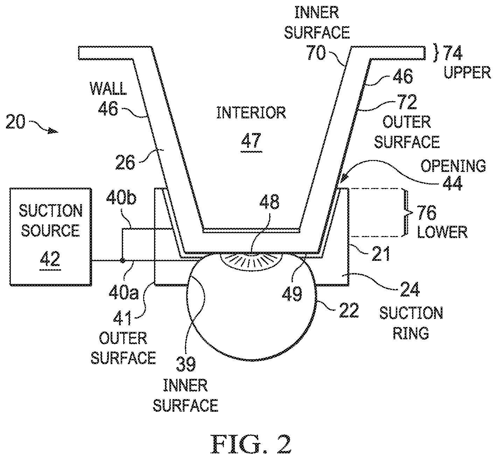

Patient interface 20 interfaces with the cornea of eye 22 to couple eye 22 to laser device 15. Patient interface 20 serves to maintain eye 22 in place so the laser beam can treat eye 22. Patient interface 20 is described in more detail with reference to FIGURE 2.

FIGURE 2 illustrates an example of patient interface 20 that may be used to stabilize the position of eye 22 relative to ophthalmic surgical system 10 of FIGURE 1, according to certain embodiments. In certain embodiments, patient interface 20 includes an attachment portion 21 and an interface portion 26. Interface portion 26 comes into contact with the cornea of eye 22, typically at the surgical site. Attachment portion 21 couples interface portion 26 to eye 22. Attachment portion 21 and interface portion 26 may be formed as one piece or may be formed as two or more separate pieces that can be removably coupled to each other.

Attachment portion 21 couples patient interface 20 to eye 22 in any suitable manner, e.g., via suction or mechanical attachment. In the example, attachment portion 21 comprises suction ring 24. Suction ring 24 has a substantially annular shape (e.g., perpendicular diameter axes are equivalent within a 20% or 10% deviation) with a ring axis, an inner surface 39, and an outer surface 41. Inner surface defines an opening 44. Suction ring 24 includes one or more evacuation channels 40 (40a, 40b) coupled to a suction source 42. Suction source 42 provides suction force. An evacuation channel 40 allows for suction to couple patient interface 20 to eye 22. In the example, the suction force through evacuation channel 40a affixes suction ring 24 to eye 22, and/or the suction force through evacuation channel 40b secures interface portion 26 to suction ring 24.

Interface portion 26 has a substantially frustoconical or cylindrical shape (e.g., perpendicular diameter axes are equivalent within a 20% or 10% deviation) with a conical or cylindrical axis and a cone wall 46 that defines a cone interior 47. Interface portion 26 is shaped to at least partially fit within opening 44. Cone wall 46 has an inner surface 70 and an outer surface 72, where inner surface 70 defines cone interior 47. Cone wall 46 has an upper portion 74 and a lower portion 76. Upper portion 74 typically couples patient interface 20 to system 10, and lower portion 76 includes or is coupled to contact portion 48. Contact portion 48 is translucent or transparent to the laser beam and has an abutment face 49 that comes into contact with the cornea of eye 22. Abutment face 49 is generally disposed outwardly from the surgical site. In certain embodiments, abutment face 49 is planar and forms a planar area on the cornea, which may define the xy-plane. In other embodiments, abutment face 49 is not planar, e.g., may be convex or concave.

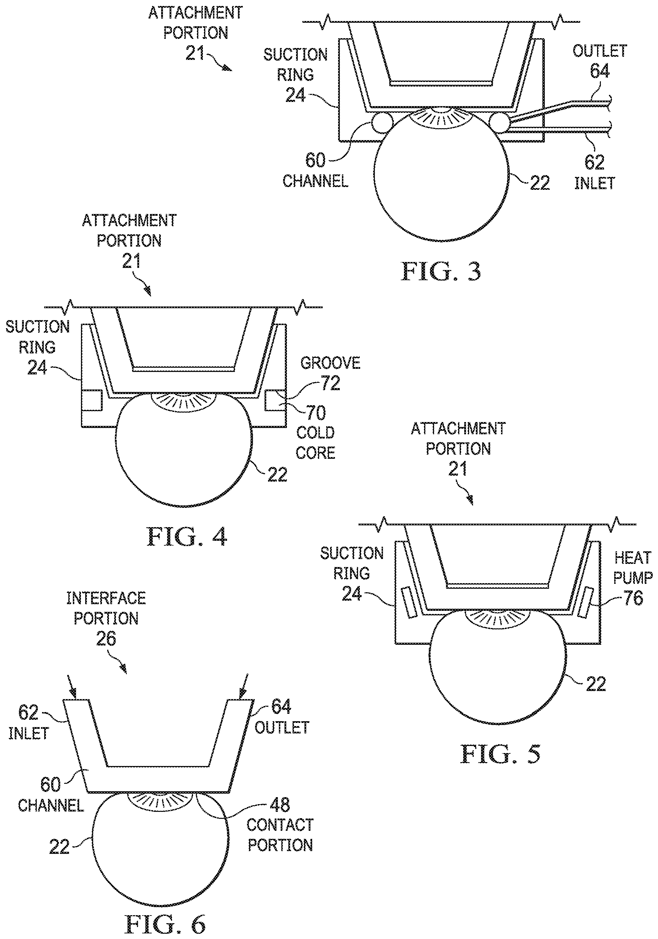

Patient interface 20 has one or more structures that direct heat away from the cornea. The structures may have any suitable configuration. Examples of such structures include: (a) one or more channels that transport fluid that carries heat away from the cornea; (b) a cold core that transports heat away from the cornea; and (c) a heat pump that cools the cornea. In certain embodiments, attachment portion 21 may include one or more of the structures to decrease the temperature of the cornea. In other embodiments, interface portion 26 may include one or more of the structures to decrease the temperature of the cornea. In yet other embodiments, attachment portion 21 and interface portion 26 may include one or more of the structures to decrease the temperature of the cornea. Examples of the structures are described in more detail with reference to FIGURES 3 to 9.

FIGURE 3 illustrates an example of attachment portion 21 of patient interface 20 of FIGURE 2 that transfers heat away from the cornea of eye 22. In the example, attachment portion 21 comprises suction ring 24 that has a channel 60 with an inlet 62 and an outlet 64. Channel 60 is disposed near eye 22 when suction ring 24 is affixed to eye. Channel 60 may have any suitable size or shape. In the example, channel 60 is annular, e.g., an annular ring, where the ring has an inner diameter equal to or slightly larger (e.g., 0 to 10 millimeters (mm)) than the inner diameter of suction ring 24. The path through channel 60 may have any suitable diameter, e.g., a diameter between 0 to 10 microns.

In an example of operation, channel 60 receives fluid through inlet 62. Any suitable biocompatible cooling or cooled fluid (e.g., liquid or gas) may be used, e.g., cooled water, saline solution, or other fluid; a compressed fluid; or a non-toxic refrigerant fluid. The fluid flows through channel 60 and out from outlet 64, transferring heat away from the cornea of eye 22.

FIGURE 4 illustrates another example of attachment portion 21 of patient interface 20 of FIGURE 2 that transfers heat away from the cornea of eye 22. In the example, attachment portion 21 has a cold core 70 that can be cooled to transfer heat away from the cornea. Cold core 70 may comprise a biocompatible cooling or cooled material (e.g., liquid, gas, gel, or solid) that absorbs heat, e.g., the biocompatible cooling or cooled fluids listed with reference to FIGURE 3 in liquid, gas, gel, or solid form; or a cooled metal or other solid. In some embodiments, cold core 70 may comprise a housing that houses the material.

Cold core 70 may have any suitable size or shape. In the example, a cold core 70 is annular with an inner diameter equal to or slightly larger than (e.g., 0 to 10 mm) the outer diameter of suction ring 24, such that cold core 70 can placed partially or completely into a receptable, such as a groove 72 of suction ring 24 of attachment portion 21. Cold core 70 may have a detachable portion that can be opened to increase the diameter of core 70 to fit around suction ring 26 and then closed to secure core 70 around suction ring 26. In an example of operation, groove 72 of suction ring 24 receives cold core 70, which transfers heat away from the cornea of eye 22.

FIGURE 5 illustrates another example of attachment portion 21 of patient interface 20 of FIGURE 2 that transfers heat away from the cornea of eye 22. In the example, attachment portion 21 has a heat pump 76 that transfers heat away from the cornea of eye 22. Any suitable heat pump 76 may be used. Examples of heat pump 76 include a Peltier cooler, thermoelectric heat pump, or other solid-state active heat pump that transfers heat.

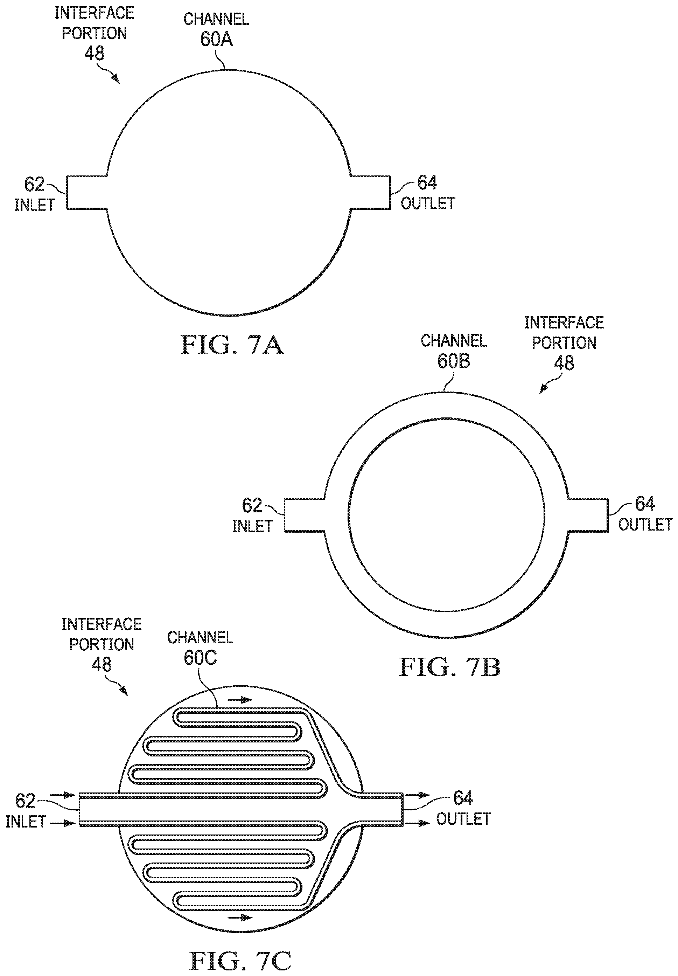

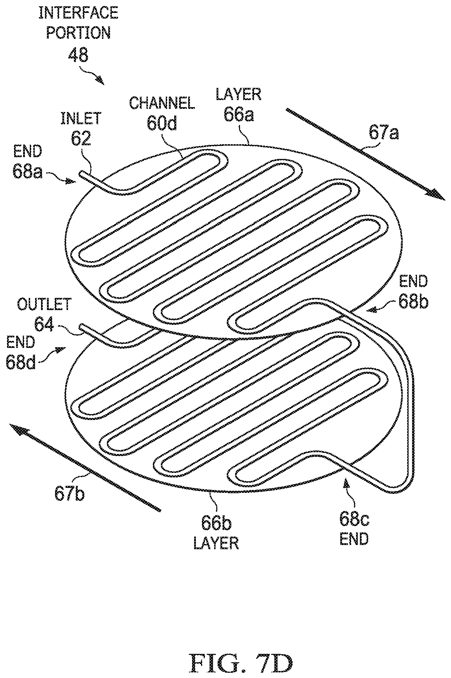

FIGURES 6 through 7D illustrate examples of interface portion 26 of patient interface 20 of FIGURE 2 that transfer heat away from the cornea of eye 22. FIGURE 6 shows a side view of channel 60; FIGURES 7A through 7C show top views of channels 60a, 60b, 60c; and FIGURE 7D shows a perspectival view of channel 60d. In the examples, interface portion 26 has a channel 60 (60a, 60b, 60c, 60d) with an inlet 62 and an outlet 64. Channel 60a is a chamber that may take up most (e.g., 75 to 90 or more than 90 %) of the area of contact portion 48. Channel 60b has a plurality of paths from inlet 62 to outlet 64. The plurality of paths may have any suitable arrangement. In the illustrated example, two paths surround the area of contact portion 48. In other examples, one or more paths may cross through the area of contact portion 48.

Channel 60c exhibits a raster pattern through contact portion 48. A raster pattern scans an area in lines that travel from a first side of the pattern to an opposite side and then back to the first side. The raster pattern of channel 60c may have any suitable configuration. In the illustrated example, a path follows a raster pattern through approximately half the area of contact portion 48, and another path follows a raster pattern through approximately the other half. In another example, one path may follow a raster pattern through most (e.g., 75 to 90 or more than 90 %) of the area. In yet another example, three or more paths may follow a raster pattern through most of the area. In yet another example, the lines may be closer together or farther apart.

Channel 60d is disposed within a plurality of layers 66 (66a, 66b) of contact portion 48. In the example, fluid enters layer 66a at an end 68a and exits at an end 68b (e.g., the opposite end) of layer 66a in a direction 67a. The fluid then enters layer 66b at an end 68c that is closer to end 68b of layer 66a and exits at an end 66d (e.g., the opposite end) of layer 66b that is closer to end 68a of layer 66a in a direction 67b that is substantially opposite to direction 67a (e.g., parallel within 40 degrees). Channel 60d is arranged such that cooler portion of layer 66a, near end 68a where the cooler fluid first enters, is disposed outwardly from the warmer portion of layer 66b, near end 68d where the warmer fluid exits, such that the cooler portion counteracts the warmer portion. Channel 60d may have any suitable pattern. In the example, channel 60d has a raster pattern, but may have any other suitable pattern, e.g., a pattern of channels 60a or 60b.

In an example of operation, channel 60 receives fluid through inlet 62. The fluid flows through channel 60, through contact portion 48 of interface 20, and out from outlet 64, transferring heat away from the cornea of eye 22. In the embodiments, channel 60 and/or the fluid may be in the path of the laser beam. Accordingly, design and/or operation of patient interface 20 and/or system 10 may need to take into account and compensate for how channel 60 and/or the fluid affect the laser beam. For example, the refractive indices of channel 60 and/or the fluid may need to be considered.

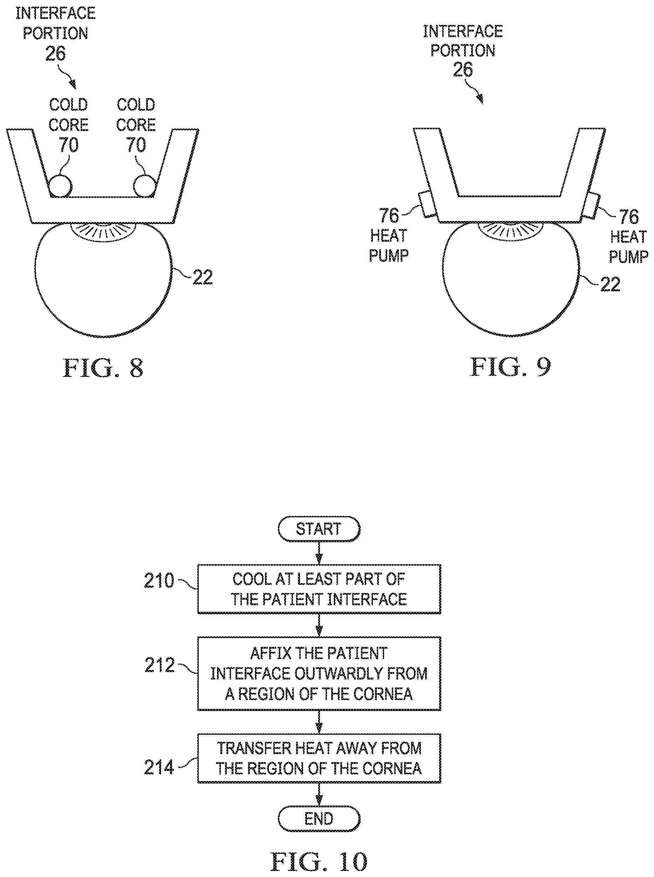

FIGURE 8 illustrates another example of interface portion 26 of patient interface 20 of FIGURE 2 that transfers heat away from the cornea of eye 22. In the example, interface portion 26 has a cold core 70 placed outwardly from contact portion 48 of patient interface 20. Cold core 70 can be cooled to transfer heat away from the cornea. Cold core 70 may have any suitable size or shape. In the example, a cold core 70 is annular or circular with an outer diameter the equal to or slightly smaller than (e.g., 0 to 10 mm) the inner diameter of interface portion 26. In an example of operation, interface portion 26 receives cold core 70, which transfers heat away from the cornea of eye 22.

FIGURE 9 illustrates another example of interface portion 26 of patient interface 20 of FIGURE 2 that transfers heat away from the cornea of eye 22. In the example, interface portion 26 has a heat pump 76 that transfers heat away from the cornea of eye 22. Heat pump 76 may be placed at or coupled to any suitable location of patient interface 20 (e.g., interface portion 26 and/or suction ring 24), such as a location where it does not interfere with the laser beam. In the example, heat pump 76 is coupled to interface portion 26, but not in contact with eye 22. In other examples, heat pump 76 may be placed where it is in contact with eye 22, such as coupled to a surface of patient interface 20 that is proximate to or touches eye 22. Any suitable heat pump 76 may be used, e.g., the heat pumps described with reference to FIGURE 5.

FIGURE 10 illustrates an example of a method for cooling the cornea during an ophthalmic procedure that may be used with patient interface 20 of FIGURE 2, according to certain embodiments. The method begins at step 210, where at least part of patient interface 20 is cooled. The cooled part may be attachment portion 21 and/or interface portion 26. In certain embodiments, the part may comprise a biocompatible cooling or cooled material (e.g., liquid, gas, gel, or solid) that absorbs heat, e.g., the materials listed with reference to FIGURE 4. Patient interface 20 is coupled outwardly from a region of the cornea at step 212. The cooled part of patient interface 20 transfers heat away from the region of the cornea at step 214 to cool the region. The method ends.

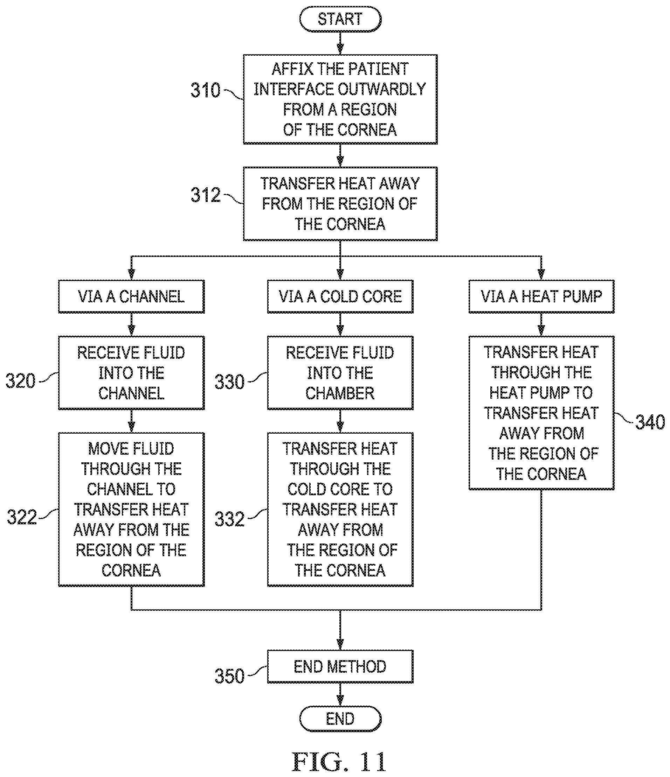

FIGURE 11 illustrates another example of a method for cooling the cornea during an ophthalmic procedure that may be used with patient interface 20 of FIGURE 2, according to certain embodiments. The method begins at step 310, where patient interface 20 is coupled outwardly from a region of the cornea. Heat is transferred away from the region of the cornea at step 312. Depending on the type of patient interface 20, heat may be transferred via a channel, cold core, or heat pump.

Heat is transferred via a channel at steps 320 and 322. Patient interface receives fluid into the channel at step 320. The fluid is moved through the channel at step 322 to transfer heat away from the region. Heat is transferred via a cold core at steps 330 and 332. Patient interface receives a cold core into a chamber or groove at step 330. The cold core transfers heat away from the region at step 332. Heat is transferred via a heat pump at step 340. The heat pump transfers heat away from the region at step 340. After transferring heat, the method ends at step 350.

A component (such as computer 30) of the systems and apparatuses disclosed herein may include an interface, logic, and/or memory, any of which may include computer hardware and/or software. An interface can receive input to the component and/or send output from the component, and is typically used to exchange information between, e.g., software, hardware, peripheral devices, users, and combinations of these. A user interface is a type of interface that a user can utilize to communicate with (e.g., send input to and/or receive output from) a computer. Examples of user interfaces include a display, Graphical User Interface (GUI), touchscreen, keyboard, mouse, gesture sensor, microphone, and speakers.

Logic can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by an electronic device to perform operations. Examples of computer software include a computer program, application, and operating system.

A memory can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, or the operations of the systems and apparatuses may be performed by more, fewer, or other components, as apparent to those skilled in the art. As another example, modifications may be made to the methods disclosed herein. The methods may include more, fewer, or other steps, and the steps may be performed in any suitable order, as apparent to those skilled in the art.

To aid the Patent Office and readers in interpreting the claims, Applicants note that they do not intend any of the claims or claim elements to invoke 35 U.S.C. §112(f), unless the words "means for" or "step for" are explicitly used in the particular claim. Use of any other term (e.g., "mechanism," "module," "device," "unit," "component," "element," "member," "apparatus," "machine," "system," "processor," or "controller") within a claim is understood by the applicants to refer to structures known to those skilled in the relevant art and is not intended to invoke 35 U.S.C. §112(f).

## Claims

1. A patient interface (20) for an ophthalmic laser system, comprising:
an interface portion (26) comprising:
a transmissive portion configured to allow a laser beam through to a cornea of an eye (22) to perform an ophthalmic procedure; and
an interface wall disposed outwardly from the transmissive portion; and an attachment portion (21) comprising a suction ring (24) and a cold core (70), the attachment portion (21) being configured to:
couple the interface portion (26) to a region of the cornea of the eye (22) to allow the laser beam through to the cornea to perform the ophthalmic procedure; and
receive the cold core (70) to decrease a temperature of the region during the ophthalmic procedure, the cold core (70) comprising a detachable portion being configured to:
open to increase a diameter of the cold core (70) to fit around the suction ring (24); and
close to couple the cold core (70) around the suction ring (24).

2. The patient interface (20) of Claim 1:
the attachment portion (21) comprising one or more channels (60); and
the attachment portion (21) being configured to decrease the temperature of the region by receiving fluid through the one or more channels (60) to transfer heat away from the region.

3. The patient interface (20) of Claim 1:
the attachment portion (21) comprising a heat pump (76) configured to transfer heat; and
the attachment portion (21) being configured to decrease the temperature of the region by transferring heat, by the heat pump (76), away from the region.

4. The patient interface (20) of Claim 1, the cold core (70) comprising a cooling material that absorbs heat.

5. The patient interface (20) of Claim 4, the cooling material comprising a cooling gel.

6. The patient interface (20) of Claim 1:
the suction ring (24) having a groove (72); and
the attachment portion (21) being configured to receive the cold core (70) via the groove (72) of the suction ring (24).

7. A patient interface (20) for an ophthalmic laser system, comprising:
an interface portion (26) comprising:
a transmissive portion comprising a channel (60) comprising a plurality of layers (66a, 66b) and being configured to convey a fluid, the plurality of layers (66a, 66b) comprising:
a first layer (66a) comprising a first entrance of the channel (60) and a first exit of the channel (60), the channel (60) configured to convey the fluid from the first entrance of the channel (60) to the first exit of the channel (60); and
a second layer (66b) disposed outwardly from the first layer (66a), the second layer (66b) comprising a second entrance of the channel (60) and a second exit of the channel (60), the channel (60) configured to convey the fluid from the first exit of the channel (60) to the second entrance of the channel (60), the channel (60) configured to convey the fluid from the second entrance of the channel (60) to the second exit of the channel (60), the transmissive portion configured to:
allow a laser beam through to a cornea of an eye (22) to perform an ophthalmic procedure at a region of the cornea; and
convey the fluid through the channel (60) to decrease a temperature of the region during the ophthalmic procedure; and
an interface wall disposed outwardly from the transmissive portion; and
an attachment portion (21) configured to couple the interface portion (26) to the region of the cornea of the eye (22) to allow the laser beam through to the cornea to perform the ophthalmic procedure.

8. The patient interface (20) of Claim 7:
the attachment portion (21) comprising a receptacle configured to receive a cold core (70); and
the attachment portion (21) being configured to decrease the temperature of the region by allowing the cold core (70) to transfer heat away from the region.

9. The patient interface (20) of Claim 7:
the attachment portion (21) comprising a heat pump (76) configured to transfer heat; and
the attachment portion (21) being configured to decrease the temperature of the region by transferring heat, by the heat pump (76), away from the region.

10. The patient interface (20) of Claim 7, the attachment portion (21) having an annular shape configured receive the interface wall of the interface portion (26) to removably couple the interface portion (26) to the attachment portion (21).

11. The patient interface (20) of Claim 7, the interface portion (26) and the attachment portion (21) being formed as one piece.

12. The patient interface (20) of Claim 7, the second entrance of the channel (60) being proximate to the first exit of the channel (60), the second entrance of the channel (60) being proximate to the first entrance of the channel (60).

## Patentansprüche

1. Patientenschnittstelle (20) für ein ophthalmisches Lasersystem, umfassend:
einen Schnittstellenabschnitt (26) umfassend:
einen durchlässigen Abschnitt, dafür gestaltet, einen Laserstrahl zu einer Hornhaut eines Auges (22) durchzulassen, um einen ophthalmischen Eingriff durchzuführen; und
eine Schnittstellenwand, die außerhalb des durchlässigen Teils angeordnet ist; und
einen Befestigungsabschnitt (21), der einen Saugring (24) und einen kalten Kern (70) umfasst, wobei der Befestigungsabschnitt (21) gestaltet ist zum:
Koppeln des Schnittstellenabschnitts (26) an einen Bereich der Hornhaut des Auges (22), um den Laserstrahl zu der Hornhaut durchzulassen, um den ophthalmischen Eingriff durchzuführen; und
Aufnehmen des kalten Kerns (70), um eine Temperatur des Bereichs während des ophthalmischen Eingriffs zu verringern, wobei der kalte Kern (70) einen abnehmbaren Abschnitt umfasst, der gestaltet ist zum:
Öffnen zum Vergrößern eines Durchmessers des kalten Kerns (70) zum Passen um den Saugring (24); und
Schließen zum Koppeln des kalten Kerns (70) um den Saugring (24).

2. Patientenschnittstelle (20) nach Anspruch 1:
wobei der Befestigungsabschnitt (21) einen oder mehrere Kanäle (60) umfasst; und
der Befestigungsabschnitt (21) dazu gestaltet ist, die Temperatur des Bereichs durch Aufnehmen von Fluid durch den einen oder die mehreren Kanäle (60) zu verringern, um Wärme von dem Gebiet abzuführen.

3. Patientenschnittstelle (20) nach Anspruch 1:
wobei der Befestigungsabschnitt (21) eine Wärmepumpe (76) umfasst, die zum Übertragen von Wärme gestaltet ist; und
der Befestigungsabschnitt (21) dazu gestaltet ist, die Temperatur des Bereichs durch Abführen von Wärme durch die Wärmepumpe (76) aus dem Gebiet zu verringern.

4. Patientenschnittstelle (20) nach Anspruch 1, wobei der kalte Kern (70) ein Kühlmaterial umfasst, das Wärme absorbiert.

5. Patientenschnittstelle (20) nach Anspruch 4, wobei das Kühlmaterial ein Kühlgel umfasst.

6. Patientenschnittstelle (20) nach Anspruch 1:
wobei der Saugring (24) eine Nut (72) aufweist; und
der Befestigungsabschnitt (21) dazu gestaltet ist, den kalten Kern (70) über die Nut (72) des Saugrings (24) aufzunehmen.

7. Patientenschnittstelle (20) für ein ophthalmisches Lasersystem, umfassend:
einen Schnittstellenabschnitt (26) umfassend:
einen durchlässigen Abschnitt, der einen Kanal (60) umfasst, der mehrere Schichten (66a, 66b) umfasst und
dazu gestaltet ist, ein Fluid zu befördern, wobei die mehreren Schichten (66a, 66b) umfassen:
eine erste Schicht (66a), die einen ersten Eingang des Kanals (60) und einen ersten Ausgang des Kanals (60) umfasst, wobei der Kanal (60) dazu gestaltet ist, das Fluid von dem ersten Eingang des Kanals (60) zu dem ersten Ausgang des Kanals (60) zu befördern; und
eine zweite Schicht (66b), die außerhalb der ersten Schicht (66a) angeordnet ist, wobei die zweite Schicht (66b) einen zweiten Eingang des Kanals (60) und einen zweiten Ausgang des Kanals (60) umfasst, wobei der Kanal (60) dazu gestaltet ist, das Fluid von dem ersten Ausgang des Kanals (60) zu dem zweiten Eingang des Kanals (60) zu befördern, wobei der Kanal (60) dazu gestaltet ist, das Fluid von dem zweiten Eingang des zweiten Kanals (60) zu dem zweiten Ausgang des Kanals (60) zu befördern, wobei der durchlässige Teil gestaltet ist zum:
Durchlassen eines Laserstrahls zu einer Hornhaut eines Auges (22), um einen ophthalmischen Eingriff an einem Bereich der Hornhaut durchzuführen; und
Befördern des Fluids durch den Kanal (60), um eine Temperatur des Bereichs während des ophthalmischen Eingriffs zu verringern; und
eine Schnittstellenwand, die außerhalb des durchlässigen Teils angeordnet ist; und
einen Befestigungsabschnitt (21), gestaltet zum Koppeln des Schnittstellenabschnitts (26) an den Bereich der Hornhaut des Auges (22), um den Laserstrahl zu der Hornhaut durchzulassen, um den ophthalmischen Eingriff durchzuführen.

8. Patientenschnittstelle (20) nach Anspruch 7:
wobei der Befestigungsabschnitt (21) eine Aufnahme umfasst, die dazu gestaltet ist, einen kalten Kern (70) aufzunehmen; und
wobei der Befestigungsabschnitt (21) dazu gestaltet ist, die Temperatur des Bereichs zu verringern, indem dem kalten Kern (70) ermöglicht wird, Wärme von dem Gebiet abzuführen.

9. Patientenschnittstelle (20) nach Anspruch 7:
wobei der Befestigungsabschnitt (21) eine Wärmepumpe (76) umfasst, die zum Übertragen von Wärme gestaltet ist; und
der Befestigungsabschnitt (21) dazu gestaltet ist, die Temperatur des Bereichs durch Abführen von Wärme durch die Wärmepumpe (76) aus dem Gebiet zu verringern.

10. Patientenschnittstelle (20) nach Anspruch 7, wobei der Befestigungsabschnitt (21) eine ringförmige Form aufweist, die dazu gestaltet ist, die Schnittstellenwand des Schnittstellenabschnitts (26) aufzunehmen, um den Schnittstellenabschnitt (26) entfernbar an den Befestigungsabschnitt (21) zu koppeln.

11. Patientenschnittstelle (20) nach Anspruch 7, wobei der Schnittstellenabschnitt (26) und der Befestigungsabschnitt (21) als ein Stück gebildet sind.

12. Patientenschnittstelle (20) nach Anspruch 7, wobei der zweite Eingang des Kanals (60) nahe dem ersten Ausgang des Kanals (60) angeordnet ist und der zweite Eingang des Kanals (60) nahe dem ersten Eingang des Kanals (60) angeordnet ist.

## Revendications

1. Interface patient (20) pour système laser ophtalmique, comprenant :
une partie d'interface (26) comprenant :
une partie transmissive configurée pour permettre à un faisceau laser à travers la cornée d'un œil (22) d'effectuer une intervention ophtalmique ; et
une paroi d'interface disposée à l'extérieur de la partie transmissive ; et
une partie de fixation (21) comprenant un anneau d'aspiration (24) et un noyau froid (70), la partie de fixation (21) étant configurée pour :
accoupler la partie interface (26) à une région de la cornée de l'œil (22) pour permettre au faisceau laser de traverser la cornée pour effectuer l'intervention ophtalmique ; et
recevoir le noyau froid (70) pour diminuer une température de la région au cours de l'intervention ophtalmique, le noyau froid (70) comprenant une partie détachable étant configurée pour :
s'ouvrir pour augmenter un diamètre du noyau froid (70) afin qu'il s'ajuste autour de l'anneau d'aspiration (24) ; et
se fermer pour accoupler le noyau froid (70) autour de l'anneau d'aspiration (24).

2. Interface patient (20) selon la revendication 1 :
la partie de fixation (21) comprenant un ou plusieurs canaux (60) ; et
la partie de fixation (21) étant configurée pour diminuer la température de la région en recevant un fluide à travers le ou les canaux (60) afin d'éloigner la chaleur de la région.

3. Interface patient (20) selon la revendication 1 :
la partie de fixation (21) comprenant une pompe à chaleur (76) configurée pour transférer la chaleur ; et
la partie de fixation (21) étant configurée pour diminuer la température de la région en éloignant la chaleur, par la pompe à chaleur (76), de la région.

4. Interface patient (20) selon la revendication 1, le noyau froid (70) comprenant un matériau de refroidissement qui absorbe la chaleur.

5. Interface patient (20) selon la revendication 4, le matériau de refroidissement comprenant un gel de refroidissement.

6. Interface patient (20) selon la revendication 1 :
l'anneau d'aspiration (24) présentant une rainure (72) ; et
la partie de fixation (21) étant configurée pour recevoir le noyau froid (70) par l'intermédiaire de la rainure (72) de l'anneau d'aspiration (24).

7. Interface patient (20) pour système laser ophtalmique, comprenant :
une partie d'interface (26) comprenant :
une partie transmissive comprenant un canal (60) comprenant une pluralité de couches (66a, 66b) et étant configurée pour transporter un fluide, la pluralité de couches (66a, 66b) comprenant :
une première couche (66a) comprenant une première entrée du canal (60) et une première sortie du canal (60), le canal (60) étant configuré pour transporter le fluide depuis la première entrée du canal (60) jusqu'à la première sortie du canal (60) ; et
une seconde couche (66b) disposée à l'extérieur de la première couche (66a), la seconde couche (66b) comprenant une seconde entrée du canal (60) et une seconde sortie du canal (60), le canal (60) étant configuré pour transporter le fluide de la première sortie du canal (60) à la seconde entrée du canal (60), le canal (60) étant configuré pour transporter le fluide de la seconde entrée du canal (60) à la seconde sortie du canal (60), la partie transmissive étant configurée pour :
laisser un faisceau laser traverser la cornée d'un œil (22) pour effectuer une intervention ophtalmique au niveau d'une région de la cornée ; et
transporter le fluide à travers le canal (60) pour diminuer une température de la région pendant l'intervention ophtalmique ; et
une paroi d'interface disposée à l'extérieur de la partie transmissive ; et
une partie de fixation (21) configurée pour accoupler la partie interface (26) à la région de la cornée de l'œil (22) pour permettre au faisceau laser de traverser la cornée pour effectuer l'intervention ophtalmique.

8. Interface patient (20) selon la revendication 7 :
la partie de fixation (21) comprenant un réceptacle configuré pour recevoir un noyau froid (70) ; et
la partie de fixation (21) étant configurée pour diminuer la température de la région en permettant au noyau froid (70) d'éloigner la chaleur de la région.

9. Interface patient (20) selon la revendication 7 :
la partie de fixation (21) comprenant une pompe à chaleur (76) configurée pour transférer la chaleur ; et
la partie de fixation (21) étant configurée pour diminuer la température de la région en éloignant la chaleur, par la pompe à chaleur (76), de la région.

10. Interface patient (20) selon la revendication 7, la partie de fixation (21) ayant une forme annulaire configurée pour recevoir la paroi d'interface de la partie d'interface (26) pour accoupler de manière détachable la partie d'interface (26) à la partie de fixation (21).

11. Interface patient (20) selon la revendication 7, la partie d'interface (26) et la partie de fixation (21) étant formées d'une seule pièce.

12. Interface patient (20) selon la revendication 7, la seconde entrée du canal (60) étant proche de la première sortie du canal (60), la seconde entrée du canal (60) étant proche de la première entrée du canal (60).
